# EUROPEAN PATENT SPECIFICATION

(11) **EP 1 034 737 B1**
(45) Date of publication and mention of the grant of the patent: **19.05.2004**
(21) Application number: 99104535.2
(22) Date of filing: 06.03.1999
(51) Int. Cl.: A61B 5/028

(54) **Injection channel for a blood vessel catheter**
Injektionskanal für einen Blutgefässkatheter
Canal d'injection pour cathéter des vaisseaux sanguins

(43) Date of publication of application: 13.09.2000
(73) Proprietor: Pulsion Medical Systems AG, 81829 München (DE)
(72) Inventor: Schmalberger, Rainer c/oPulsion Verw. GmbH & Co., 81675 München (DE); Fähle, Matthias c/oPulsion Verw. GmbH & Co., 81675 München (DE); Borg, Ulf c/oPulsion Verw. GmbH & Co., 81675 München (DE); Pfeiffer, Ulrich c/oPulsion Verw. GmbH & Co., 81675 München (DE)
(74) Representative: Kehl, Günther, Dipl.-Phys.

(56) References cited:
- EP-A- 0 900 545
- CA-A- 2 246 563
- US-A- 4 730 623
- US-A- 5 009 234
- US-A- 5 595 181

## Description

### Background of the Invention

Cardiac Output (CO) and circulating blood volume are very important parameters to make statements about the cordition of clinically ill patients. Measuring these parameters is a very important tool in intensive care as well as in research. Normally measurements are done on critically ill patients in head surgical treatment and for pharmacological management strategies.

The invention is a device to detect the start and end of injection as well as the injectate temperature during thermodilution determination of cardiocirculatory parameters and intra- and extravascular volumes. When employing the method of thermodilution for cardiac output determination, a liquid indicator colder than the blood temperature is injected into the right atrium or the superior or inferior vena cava. After a period of time, depending on the blood flow through the head and pulmonary circulation, a temperature drop can be detected in the femoral artery. By plotting the temperature drop over time the area under the resulting curve can be used to determine cardiac output. To calculate cardiac output it is crucial to know the exact temperature of the blood and the injected liquid. The blood temperature is measured with an indwelling thermistor in the femoral artery. This sensor can be a temperature dependent resistor with a negative coefficient (NTC).

The temperature of the injected liquid is also measured with a resistor of the same type. This resistor is in thermal contact with the liquid in the injection channel. Hence the temperature of the injected liquid can be measured during injection. In order to reuse the sensor a thermal bridge is established between the sensor and the fluid path using a liquid impermeable material. This device is called IITS device (IITS) and is a sterile disposable item. The IITS is connected in series with the injection channel and contains the holder for the temperature sensor. Additionally, in this invention a membrane is included as an actuator of a timing device. Using defined time intervals on the indicator dilution curve allow calculation of intra- and extravascular volumes between site of injection and site of detection. However, precise detection of beginning and end of injection is necessary.

Since determining these specific time points manually is impractical (imprecise), detection of a sudden step function of the temperature in the IITS is used. A sudden change in temperature is achieved by using cold injectate through the IITS that is at room temperature before injection. Consequently the injectate must be cooled prior to injection which is a big disadvantage to the user in the intensive care unit, operating room or ambulance. To provide cold injectate imposes not only additional labor time but may also introduce measurement errors and health risk to the patient. If the injectate is removed from the cooling compartment and injection is delayed, the liquid could have warmed up and this may result in erroneous measurement. If several consecutive determinations are performed the IITS will be cooled and temperature detection deteriorates. The use of cooling set at the bedside does not adequately address the above mentioned problems. The cooling set can only provide cold injectate provided that ice is constantly replenished in the cooler. The additional cooler also represents an added expense.

A device of the kind referred to above is for example disclosed in CA-A-2 246 563. In particular, the injection channel disclosed therein includes the features of the preamble of claim 1.

Because of the disadvantages mentioned above, use of injectate at room temperature would simplify the measurement. The basic principle of thermodilution is not a problem usually the room temperature is lower than the blood temperature. If injectate at room temperature is used the problem is detection of injection since no change in temperature can be detected with the IITS already at room temperature. The solution would be another method for detection of injection. The new IITS is a device that allows detection of the injectate temperature as well as beginning and end of injection at room temperature

### 2 Description of the Invention

The invention is a device as defined in claim 1 to detect the start and the end of injection for a thennodilution measurement at room temperature (∼20°C) without using cooled or heated injectate.

### 2.1 Example 1: Simulation of the Step Function Temperature

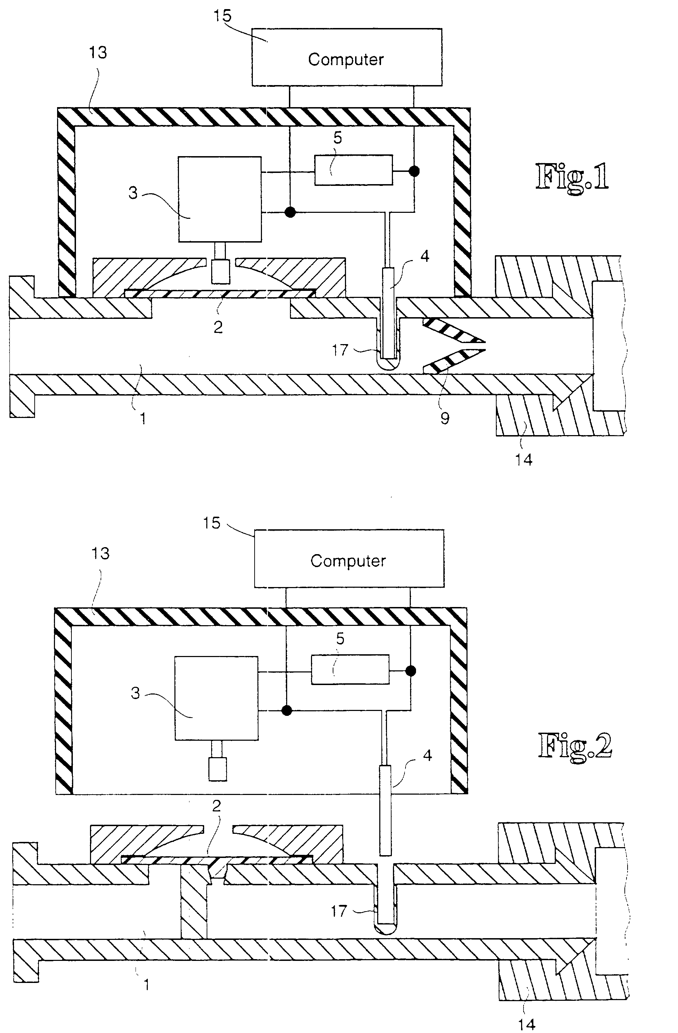

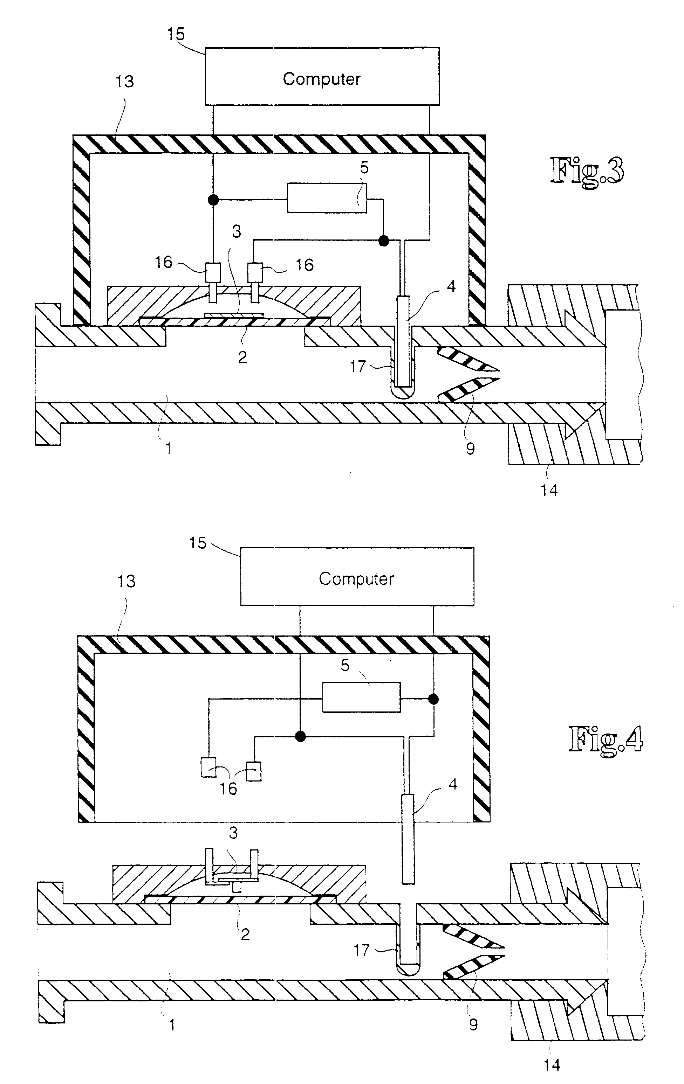

To measure the temperature of the injectate for a thermodilution measurement a temperature depending resistor is used. This temperature depending resistor (thermistor) can be a PTC (positive coefficient of temperature) or a NTC (negative coefficient of temperature). Start and end of injection is detected as step function of the resistance of the sensor branch for temperature measurement. The switch 3 and a resistor 5 can be connected parallel to the temperature depending resistor (temperature sensor 4) as shown in the embodiments of figures 1, 2, and 4 or in serial connection as shown in figure 3 to generate this step function. At the moment of injection the switch opens or closes and generates a sudden change of resistance of the overall set-up. Before beginning cf injection a deviating value of the temperature is displayed, whereby at injection the true value is displayed.

### 2.1.1 Example 1: Variation 1:

In this variation the pressure rise at injection caused by an impedance in the channel of injection is employed. This resistance is the valve (9) in figure 1. It is set-up as a check valve and prevents back flow of liquid against the mainstream (no pressure rise at injection from the catheter side). In case catheters with large cross sections are used the valve also assures that the pressure rise is still sufficient to displace the membrane 2. This membrane is displaced and actuates the switch (3). The switch, depending on set-up closes or opens. Therefore from the view of the acquisition computer the overall resistance of the whole set-up including the thermistor (4) changes. At end of injection the switch is reset due to the restoring force of the membrane (2).

Valve (9) and membrane are dimensioned, so that the valve (9) only opens when the pressure rise at injection is sufficient to displace the membrane (2) and actuates the switch (3). Switch, resistor and thermistor are in a common housing (13), that is removable. The same unit is used in figure 2 where the housing is shown separated from the injection channel.

### 2.1.2 Example 1: Variation 2:

This Variation of the invention is sketched in figure 2. It is an Inline Injectate Temperature Sensor with a membrane (2), a holder (17) for the sensor and a temperature sensor (4). In this housing the pressure rise is transformed in a mechanical displacement of the membrane (2), that is attached to the housing. The membrane actuates the switch (3). The membrane is the barrier between atmosphere and under tension. If the membrane (1) is displaced switch (3) opens or closes. It is connected to temperature sensor (4) and resistor (5). At injection from the catheter side, the pressure rise caused by the pin on the membrane (2) closing the passage to the output, displaces the membrane. This pin is the variable impedance in the injection channel, opening the passage for the liquid, dependent on pressure and hence flow rate. If the pressure is sufficient to displace the membrane the liquid can flow causing the pressure to fall, hence :his feedback keeps the pressure constant. At end of injection the restoring force of the membrane puts the pin back in position and switch (3) is reset. In this set-up the length of the pin sets the displacement of the membrane. It should be long enough, so that before it opens the passage, the switch (3) should be actuated by the displacement of the membrane. The tension of the membrane sets the opening pressure. In this variation the passage and the pin on the membrane (2) constitute the check valve in the previous variation. Liquid from the syringe side sees a large effective area, compared to liquid from the catheter side which sees only a small effective area, hence a large pressure is required to displace the membrane.

Switch (3) is connected to resistor (5) according to 2.1. The temperature sensor and the resistor are in a common removable housing (13). The output of the circuit is a cable with two wires, which is hooked up to the data acquisition device.

### 2.1.3 Example 1, Variation 3:

The sketch for this example is given in figure 3. It is similar to variation 1. The difference compared to variation is that the switch is not in housing (13). It merely is integrated in the disposable Inline Injectate Temperature Sensor device. A conductive coating on the atmospheric side of the membrane (2) and two contacts molded into the support of the membrane constitute the switch 3 in this variation. Before injection the switch is open. At injection the pressure in the IITS rises and displaces the membrane (2). This displacement makes the conductive coating of the membrane to touch the contacts and hence the switch is closed. For function of this setup it is necessary to use the circuit in which the switch 3 is connected in series to the temperature sensor as shown in figure 3, since at injection the switch is closed and not opened. The other parts of the setup are the valve with the variable impedance (9), the contacts 16 on the removable housing (13), the holder 17 for the temperature sensor, the resistor (5) and the temperature sensor.

For the setup the principle from example 1,. variation 2 could be used as well.

### 2.1.4 Example 1, Variation 4:

This variation is akin to the previous one. Its sketch is given in figure 4. The switch (3) is integrated in the IITS. Before injection, the switch (3) is closed. When injection happens the membrane is displaced and switch (3) opens. Since this setup works with opening of the switch at injection a circuit must be used in which the switch is connected parallel to the temperature sensor as shown in figure 4.

In the setup is the valve with the variable impedance (9), (16) the contacts in the removable housing (13), (5) denotes the resistor, (17) is the holder for the temperature sensor and (4) the temperature sensor. For the setup the principle from example 1, variation 2 could be used as well.

### 2.1.5 Example 1, Variation 5: Simulation of the Temperature Step Function Using a Reedswitch and a Magnet

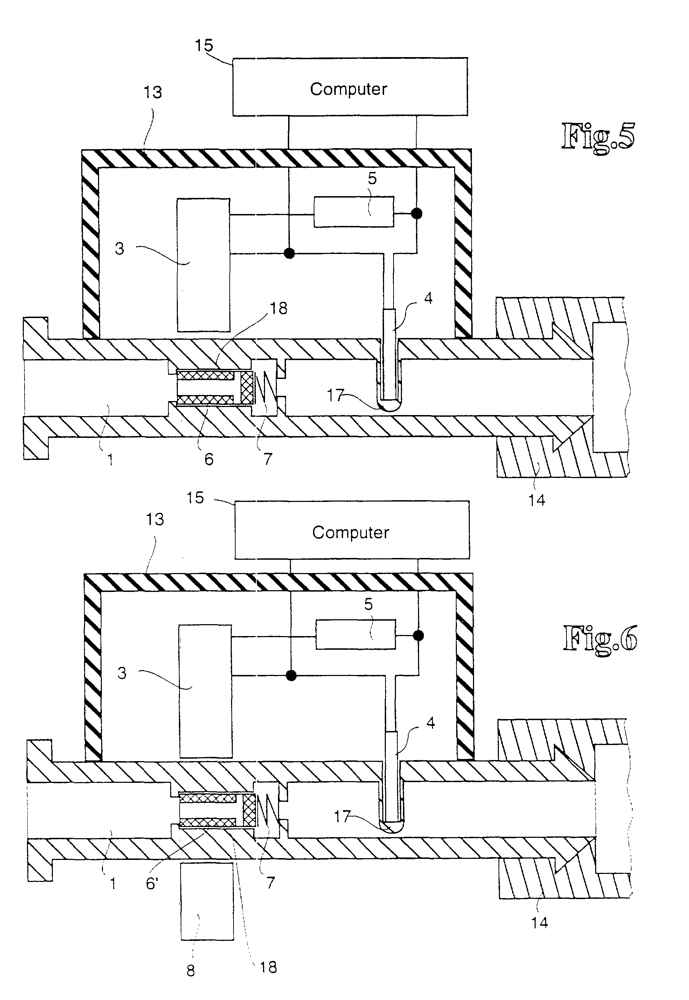

Figure 5 shows the sketch of this variation. A Reedswitch (3) is used as switch. In the injection channel there is a piston/magnet (6) made from magnetic material. It has an axial blind hole and one or more radial holes. If the piston (6) is in the proximity of the Reedswitch (3), the magnet actuates the switch. If the piston (6) is distant from the Reedswitch (3) there is no influence from the magnetic field of the piston (6).

Spring (7) pushes piston (6) in the piston guide (18). In the resting position the radial holes are closed and prevent any flow of liquid. Injection causes a pressure rise which moves the piston (6) aga nst the restoring force of the spring (7) out of the piston guide (18). If the radial holes protrude from the piston guide liquid can flow and the pressure will not continue to rise. The displacement of the piston (6) prevents the magnetic field from influencing the Reedswitch (3) and the switch (3) opens or closes. When pressure falls at erd of injection, piston (6) is forced back into the piston guide by spring (7) and the Reedswitch is reset. The change of the overall resistance of the setup is achieved by combination of Reedswitch (3) with resistor (5) and temperature sensor (4) in a common removable housing.

### 2.1.6 Example 1, Variation 6: Simulation of the Temperature Step Function using a Reedswitch, a Magnet and a Ferromagnetic Metal

This Variation is shown in Figure 6 A Reedswitch (3) is used. In the injection channel there is piston (6') made from fer omagnetic metal. It has a blind hole and one or more radial bores (see sketch). Is the piston between magnet (8) and Reedswitch the metal deflects the magnetic field from the Reedswitch (3). If the piston is not present, the magnetic field can actuate the Reedswitch. Spring (7) pushes piston (3) in the piston guide (18). In the resting position the radial holes are obstructed by the piston guide and no liquid can flow. At injection the pressure rise moves the piston (6') against the restoring force of the spring (7) out of the piston guide. Liquid flows when the radial holes in the piston protrude out of the piston guide (18). The displacement of the metal allows the magnet to open or close the Reedswitch. At end of injection the piston is pushed back in its original position by the spring (7) and the Reedswitch is reset. The change in resistance at injection is attained with the combination of Reedswitch (3) with resistor (5) and temperature sensor (4), which are in a common removable housing (13). Index (17) labels the holder for the temperature sensor (4).

### 2.1.7 Example 1, Variation 7: Simulation of the Temperature Step Function using a Reedswitch, a Magnet and a Ferromagnetic Metal

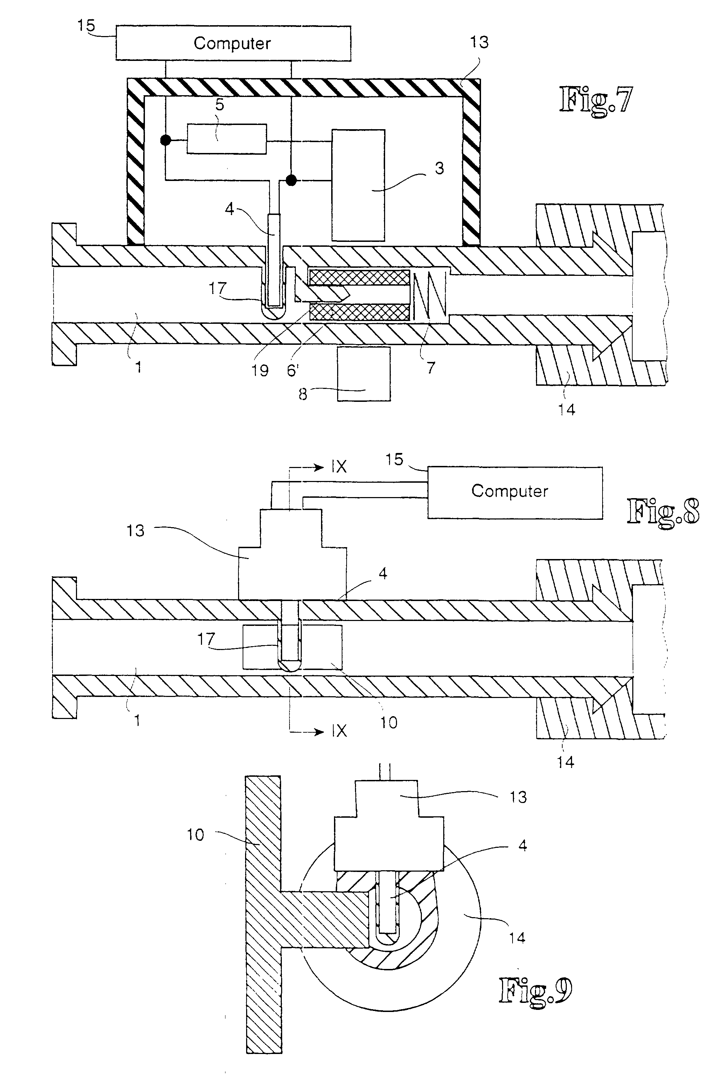

A ferromagnetic metal is used for this variation shown in figure 7. A Reedswitch (3) is used as well. In the injection channel there is piston (6') made from ferromagnetic metal. It looks like a little tube (see sketch), which is closed by pin (19) that acts like a plug. Is the piston between magnet (8) and Reedswitch the metal deflects the magnetic field from the Reedswitch (3). If the piston is not present, the magnetic field can actuate the Reedswitch. Spring (7) pushes piston (6') towards the plug (19). In the resting position the bore of the tube is obstructed by the plug and no liquid can flow. At injection the pressure rise moves the piston (6') against the restoring force of the spring (7) out of the plug (19). Liquid flows when the plug (19) doesn't close the bore anymore. The displacement of the metal allows the magnet to open or close the Reedswitch. At end of injection the piston is pushed back in its original position by the spring (7) and the Reedswitch is reset. The change in resistance at injection is attained with the combination of Reedswitch (3) with resistor (5) and temperature sensor (4), which are in a commor housing (13). Index (17) labels the holder for the temperature sensor (4).

### 2.2 Example 2: Using the Body Heat to Warm Up Injectat

If the IITS device is at body temperature (see also figure 8), there is no need to cool the injectate. The liquid inside the Inline Injectate Temperature Sensor, with the holder (17) for the temperature sensor (4), is warmed up via the Heat Contact Plate (10) made from nonsensitizing, heat conducting material that is applied to the skin of the patient. The increase in temperature depends upon the temperature of the skin, the location and on the ambient conditions. However most of the time it is significantly higher than the room temperature (on average differs more than 2°C).

The small volume of liquid in the IITS is warmed up via the heat bridge (10) which is in thermal contact to the Heat Contact Plate, to a temperature that is higher than the room temperature. At injection the injectate at room temperature causes a sudden change in temperature that can be detected by the data acquisition system. After injection the liquid in the Inline Injectate Temperature Sensor is once again heated up by the body heat of the patient, and the end of the injection can be detected.

### 2.3 Example 3: Employing Self-Heating Effect of the Temperature Sensor

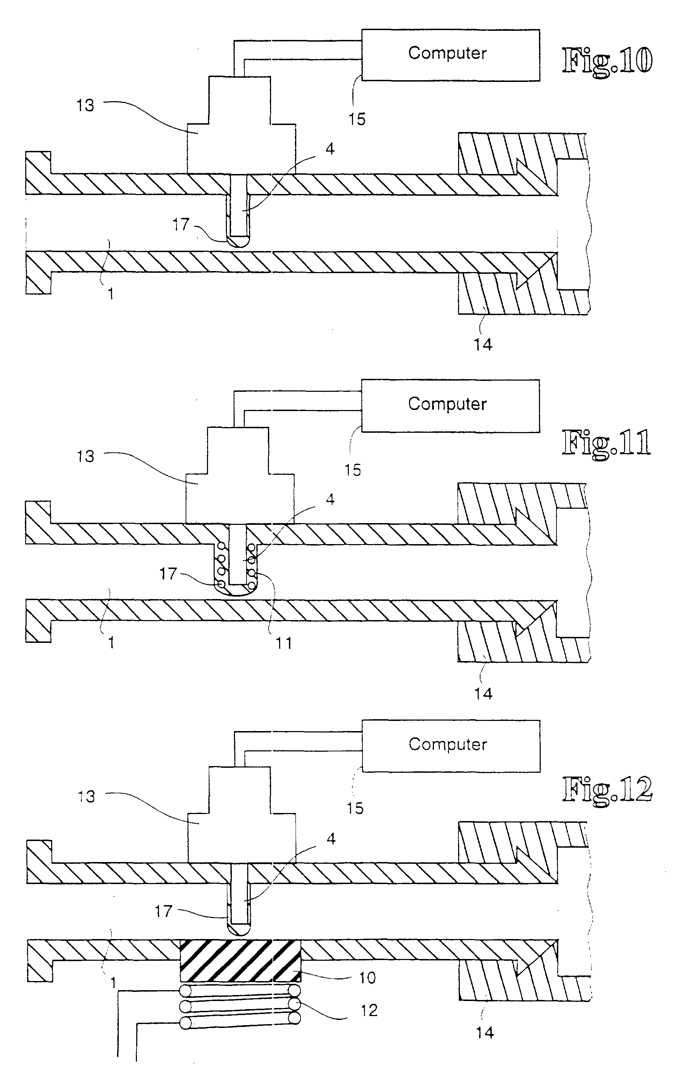

The sketch for this example is given in figure 10. In this example the temperature difference prior to injection of injectate at room temperature is produced with the help of external energy supplied by the data acquisition computer. The temperature sensor (4), a thermistor is located in a holder (17) in the IITS and operated with high and constant measurement current. The high current heats up the thermistor (4). It dissipates the heat to its surroundings, the holder and hence the static liquid the holder (17) is immersed in. The power supplied to the thermistor (4) is kept constant. The power supplied should be sufficient to cause a difference in temperature of 2°C for the static case (liquid doesn't flow, free convection) compared to the dynamic one (liquid flows, forced convection). The measured difference in temperature is created by the gradient of temperature of heat transfer. This gradient is different for the static and the dynamic case. The measured temperature is the real temperature of the injectate plus an offset due to the heating of the thermistor, which has to be accounted for. This offset also changes with the liquid used and the difference of the injectate temperature from the room temperature. At the end of injection, when there is now flow of liquid and the static case is present, the measured temperature due to the self heating of the thermistor rises and the end of injection can be detected.

### 2.4 Example 4: Heating of the Temperature Sensor

The according sketch can be found in figure 11. The difference in temperature is produced with an external heater that is supplied from a battery or the data acquisition computer. The principle is the same as in example 3, only with the difference that to get the temperature difference the temperature sensor (4) is heated with a heating element (11).

### 2.5 Example 5: Direct Heating of the Small Amount of Liquid in the Inline Injectate Temperature Sensor

The liquid in the Inline Injectate Temperature Sensor is heated by electrical means (see sketch figure 12). The necessary energy is supplied by the data acquisition computer or an external source like a battery. The electrical heating (12) is attached to the IITS. It is in thermal contact with the Heat Contact Plate (10) of the IITS. The principle is the same as the one in example 2, which uses the body heat of the patient, only with the exception, that this time the heat is produced electrically. Since the small amount of liquid in the Inline Injectate Temperature Sensor is heated, injectate at room temperature generates a sudden change in temperature at injection. After injection the liquid remaining in the Inline Injectate Temperature Sensor is heated again, causing a rise in temperature, which signals the end of the injection. Compared to example 4 and 5, this method requires more power to heat up the liquid in a sufficient time, since more heat is transferred to its surroundings. Index (4) labels the temperature sensor and (17) labels its holder.

## Claims

1. An injection channel (1) for a blood vessel catheter (14) for injecting an injectate fluid into a blood vessel of a patient for carrying out thermodilution or dye-dilution measurements in order to determine hemodynamic parameters of the patient, the injection channel (1) comprising:
a temperature sensor (4) inside the injection channel (1) for sensing the temperature of the injectate fluid passing through the injection channel (1);
a computer coupled to the temperature sensor for detecting changes of temperature readings as instants of begin and end of an injection process; **characterized by**
a flow rate and/or pressure switch (3) which is opened or closed if the flow rate/pressure in the injection channel (1) exceeds a threshold,
wherein the flow rate and/or pressure switch (3) is electrically coupled to the output of the temperature sensor (4) in such a manner that in case the flow rate/pressure inside the injection channel (1) is lower than the threshold, the readings of the temperature sensor (4) are modified to indicate a temperature which deviates from the true temperature readings and in case the flow rate/pressure inside the injection channel (1) is exceeding the threshold, the readings of the temperature sensor are switched abruptly to the true readings.

2. The injection channel (1) according to claim 1, wherein the flow rate and/or pressure switch (3) is an NO-normally open-switch and is coupled in a serial circuit with regard to the output of the temperature sensor (4).

3. The injection channel (1) according to claim 1 or 2, wherein the flow rate and/or pressure switch (3) is an NC-normally closed-switch and is coupled in a parallel circuit with regard to the output of the temperature sensor (4).

4. The injection channel (1) according to anyone of the preceding claims, wherein a resistor (5) is coupled in series to the temperature sensor (4) and wherein the flow rate and/or pressure switch (3) is connected to bypass the resistor (5).

5. The injection channel (1) according to anyone of the preceding claims, wherein a resistor (5) is coupled in parallel to the temperature sensor (4) and wherein the flow rate and/or pressure switch (3) is connected in series to the resistor (5).

6. The injection channel (1) according to anyone of the preceding claims, wherein the flow rate and/or pressure switch (3) is actuated by an elastic membrane (2) which is displaced by the influence of the fluid flow/pressure inside the injection channel (1).

7. The injection channel (1) according to anyone of the preceding claims, wherein the flow rate and/or pressure switch (3) comprises a REED-switch (8) outside the injection channel (1) being actuated by a movable magnet (8) biased by an elastic member (7) in the injection channel (1), the magnet (6) being displaced by the influence of the fluid flow/pressure inside the injection channel (1).

8. The injection channel (1) according to anyone of the preceding claims, wherein the flow rate and/or pressure switch (3) comprises a REED-switch outside the injection channel (1) and a magnet (8) outside the injection channel (1) at a side opposite to the REED-switch, the REED-switch being actuated by the influence of the movement of a movable ferromagnetic member (6') biased by an elastic member (7) in the injection channel (1) which ferromagnetic member (6') is displaced by the influence of the fluid flow/pressure inside the injection channel (1) whereby the ferromagnetic member shields the REED-switch from the magnet (8) in a first position and exposes the REED-switch to the magnet in a second position.

9. The injection channel (1) according to anyone of the preceding claims, connected to an injectate source at one side and to a blood vessel catheter (14) at the other side.

10. The injection channel (1) according to anyone of the preceding claims, wherein the flow rate and/or pressure switch (3) and the temperature sensor (4) are arranged at least partially in a separate removable housing.

11. The injection channel (1) according to anyone of the preceding claims, further comprising a throttle device (9) inside the injection channel (1) downstream of the pressure switch (3).

12. The injection channel (1) according to anyone of the preceding claims, further comprising a check valve (9) inside the injection channel (1) that opens if a certain pressure is exceeded.

## Patentansprüche

1. Injektionskanal (1) für einen Blutgefäßkatheter (14) zum Injizieren eines Injektatfluids in ein Blutgefäß eines Patienten zum Durchführen von Thermodilutions- oder Farbstoffdilutions-Messungen, um hämodynamische Parameter des Patienten zu bestimmen, wobei der Injektionskanal (1) folgendes aufweist:
einen Temperatursensor (4) innerhalb des Injektionskanals (1) zum Erfassen der Temperatur des Injektatfluids, das den Injektionskanal (1) durchströmt;
einen Computer, der mit dem Temperatursensor verbunden ist, um Änderungen von Temperaturmeßwerten als Zeitpunkte von Beginn und Ende eines Injektionsvorgangs festzustellen; **gekennzeichnet durch**
einen Strömungsrate- und/oder Druckschalter (3), welcher geöffnet oder geschlossen wird, wenn die Strömungsrate/der Druck in dem Injektionskanal (1) einen Schwellenwert überschreitet,
wobei der Strömungsrate- und/oder Druckschalter (3) elektrisch an den Ausgang des Temperatursensors (4) derart gekoppelt ist, dass, falls die Strömungsrate/der Druck innerhalb des Injektionskanals (1) niedriger als der Schwellenwert ist, die Meßwerte des Temperatursensors (4) modifiziert werden, um eine Temperatur anzuzeigen, welche von den tatsächlichen Temperaturmeßwerten abweicht, und falls die Strömungsrate/der Druck innerhalb des Injektionskanals (1) den Schwellenwert überschreitet, die Meßwerte des Temperatursensors abrupt auf die tatsächlichen Meßwerte umgeschaltet werden.

2. Injektionskanal (1) nach Anspruch 1, bei dem der Strömungsrate- und/oder Druckschalter (3) ein Arbeitskontaktschalter (NO-normally open-Schalter) ist und in bezug auf den Ausgang des Temperatursensors (4) in Serie geschaltet ist.

3. Injektionskanal (1) nach Anspruch 1 oder 2, bei dem der Strömungsrateund/oder der Druckschalter (3) ein Ruhekontaktschalter (NC-normally closed-Schalter) ist und in bezug auf den Ausgang des Temperatursensors (4) parallel geschaltet ist.

4. Injektionskanal (1) nach einem der vorhergehenden Ansprüche, bei dem ein Widerstand (5) seriell mit dem Temperatursensor (4) verbunden ist und bei dem der Strömungsrate- und/oder Druckschalter (3) den Widerstand (5) überbrückt.

5. Injektionskanal (1) nach einem der vorhergehenden Ansprüche, bei dem ein Widerstand (5) parallel zu dem Temperatursensor (4) geschaltet ist und bei dem der Strömungsrate- und/oder Druckschalter (3) in Serie zu dem Widerstand (5) geschaltet ist.

6. Injektionskanal (1) nach einem der vorhergehenden Ansprüche, bei dem der Strömungsrate- und/oder Druckschalter (3) durch eine elastische Membran (2) betätigt wird, die durch den Einfluß der Fluidströmung/des Druckes innerhalb des Injektionskanals (1) bewegt wird.

7. Injektionskanal (1) nach einem der vorhergehenden Ansprüche, bei dem der Strömungsrate- und/oder Druckschalter (3) einen REED-Schalter außerhalb des Injektionskanals (1) aufweist, der von einem durch ein elastisches Element (7) in dem Injektionskanal (1) vorgespannten bewegbaren Magnet (8) betätigt wird, wobei der Magnet (6) durch den Einfluß der Fliudströmung/des Druckes innerhalb des Injektionskanals (1) bewegt wird.

8. Injektionskanal (1) nach einem der vorhergehenden Ansprüche, bei dem der Strömungsrate- und/oder Druckschalter (3) einen REED-Schalter außerhalb des Injektionskanals (1) und einen Magnet (8) außerhalb des Injektionskanals (1) auf der gegenüberliegenden Seite des REED-Schalters aufweist, wobei der REED-Schalter durch den Einfluß der Bewegung eines bewegbaren ferromagnetischen Elements (6'), das durch ein elastisches Element (7) in dem Injektionskanal (1) vorgespannt ist, betätigt wird, welches ferromagnetische Element (6') durch den Einfluß der Fluidströmung/des Druckes innerhalb des Injektionskanals (1) bewegt wird, wodurch das ferromagnetische Element den REED-Schalter von dem Magneten (8) in einer ersten Position abschirmt und den REED-Schalter dem Magneten in einer zweiten Position exponiert.

9. Injektionskanal (1) nach einem der vorhergehenden Ansprüche, der mit einer Injektatquelle an einer Seite und einem Blutgefäßkatheter (14) an der anderen Seite verbunden ist.

10. Injektionskanal (1) nach einem der vorhergehenden Ansprüche, bei dem der Strömungsrate- und/oder Druckschalter (3) und der Temperatursensor (4) wenigstens zum Teil in einem getrennten abnehmbaren Gehäuse angeordnet sind.

11. Injektionskanal (1) nach einem der vorhergehenden Ansprüche, der außerdem eine Drosselungsvorrichtung (9) innerhalb des Injektionskanals (1) nach dem Druckschalter (3) aufweist.

12. Injektionskanal (1) nach einem der vorhergehenden Ansprüche, der zudem ein Rückschlagventil (9) innerhalb des Injektionskanals (1) aufweist, welches öffnet, wenn ein bestimmter Druck überschritten wird.

## Revendications

1. Canal d'injection (1) pour un cathéter vasculaire (14) pour injecter un liquide injectable dans le vaisseau sanguin d'un patient, pour réaliser des mesures de thermodilution ou de dilution de colorant dans le but de déterminer les paramètres hémodynamiques du patient, le canal d'injection (1) comprenant :
- un capteur de température (4) à l'intérieur du canal d'injection (1) pour capter la température du produit injecté qui circule dans le canal d'injection (1),
- un calculateur couplé au capteur de température pour détecter des valeurs de changements de température à titre d'instants de début et de fin d'un processus d'injection,
**caractérisé par** :
- un interrupteur (3) de débit et/ou de pression qui est ouvert ou fermé si le débit/la pression dans le canal d'injection (1) excède un seuil,
- dans lequel l'interrupteur (3) de débit et/ou pression est couplé électriquement à la sortie du capteur de température (4) de façon telle que, lorsque le débit/la pression dans le canal d'injection (1) est inférieur(e) au seuil, les valeurs relevées par le capteur de température (4) sont modifiées pour indiquer une température qui s'écarte des valeurs réelles de température relevées et lorsque le débit/la pression dans le canal d'injection (1) excède le seuil, les valeurs relevées par le capteur de température basculent subitement sur les valeurs réelles relevées.

2. Canal d'injection (1) selon la revendication 1, dans lequel l'interrupteur (3) de débit et/ou pression est un interrupteur normalement ouvert et est couplé dans un circuit en série avec la sortie du capteur de température (4).

3. Canal d'injection (1) selon la revendication 1 ou 2, dans lequel l'interrupteur (3) de débit et/ou pression est un interrupteur normalement fermé et est couplé dans un circuit en parallèle par rapport à la sortie du capteur de température (4).

4. Canal d'injection (1) selon l'une quelconque des revendications précédentes, dans lequel une résistance (5) est couplée en série avec le capteur de température (4), et dans lequel l'interrupteur (3) de débit et/ou pression est connecté pour mettre la résistance (5) en dérivation.

5. Canal d'injection (1) selon l'une quelconque des revendications précédentes, dans lequel une résistance (5) est couplée en parallèle avec le capteur de température (4), et dans lequel l'interrupteur (3) de débit et/ou pression est connecté en série à la résistance (5).

6. Canal d'injection (1) selon l'une quelconque des revendications précédentes, dans lequel l'interrupteur (3) de débit et/ou pression est actionné par une membrane élastique (2) qui est déplacée par l'effet du débit/de la pression du liquide dans le canal d'injection (1).

7. Canal d'injection (1) selon l'une quelconque des revendications précédentes, dans lequel l'interrupteur (3) de débit et/ou pression comprend un interrupteur à tiges "REED" (8) monté à l'extérieur du canal d'injection (1), actionné par un aimant mobile (6) rappelé par un élément élastique (7) dans le canal d'injection (1), l'aimant (6) étant déplacé sous l'effet du débit/de la pression du liquide à l'intérieur du canal d'injection (1).

8. Canal d'injection (1) selon l'une quelconque des revendications précédentes, dans lequel l'interrupteur (3) de débit et/ou pression comprend un interrupteur à tiges "REED" monté à l'extérieur du canal d'injection (1) et un aimant (8) monté à l'extérieur du canal d'injection (1) sur un côté opposé à l'interrupteur à tiges "REED", l'interrupteur à tiges "REED" étant actionné sous l'effet du mouvement d'un élément ferromagnétique mobile (6') rappelé par un élément élastique (7) dans le canal d'injection (1), ledit élément ferromagnétique (6') étant déplacé sous l'effet du débit/ de la pression du liquide dans le canal d'injection (1) de façon telle que l'élément ferromagnétique protège l'interrupteur "REED" de l'aimant (8) dans une première position et expose l'interrupteur "REED" à l'aimant dans une seconde position.

9. Canal d'injection (1) selon l'une quelconque des revendications précédentes, connecté à une source de produit d'injection d'un côté, et à un cathéter vasculaire (14) de l'autre côté.

10. Canal d'injection (1) selon l'une quelconque des revendications précédentes, dans lequel l'interrupteur (3) de débit et/ou pression et le capteur de température (4) sont agencés au moins partiellement dans un boîtier séparé amovible.

11. Canal d'injection (1) selon l'une quelconque des revendications précédentes, comprenant de plus un dispositif d'étranglement (9) dans le canal d'injection (1) en aval de l'interrupteur (3) de pression.

12. Canal d'injection (1) selon l'une quelconque des revendications précédentes, comprenant de plus un clapet antiretour (9) dans le canal d'injection (1), qui s'ouvre si une certaine pression est dépassée.
